# EUROPEAN PATENT APPLICATION

(11) **EP 4 443 154 A1**
(43) Date of publication of application: **09.10.2024**
(21) Application number: 22901216.6
(22) Date of filing: 28.11.2022
(51) Int. Cl.: G01N 27/624, G01N 33/497

(54) **DISEASE OR HEALTH STATE INSPECTION METHOD AND INSPECTION SYSTEM**

(30) Priority: 30.11.2021 JP 2021194205
(71) Applicant: Daikin Industries, Ltd., Osaka-shi, Osaka 530-0001 (JP); Matrix Cell Research Institute Inc., Ushiku-shi, Ibaraki 300-1232 (JP); Faimstech Japan, Inc., Tokyo 101-0025 (JP)
(72) Inventor: MATSUBARA, Akira, Osaka 530-0001 (JP); ARAI, Junichiro, Osaka 530-0001 (JP); KUWAMURA, Yuma, Osaka 530-0001 (JP); GOTO, Takashi, Osaka 530-0001 (JP); KUSAKABE, Moriaki, Ushiku-shi, Ibaraki 300-1232 (JP); PSUTKA, Christopher, Chiyoda-ku, Tokyo 101-0025 (JP); MIKOSHIBA, Tohru, Chiyoda-ku, Tokyo 101-0025 (JP)
(74) Representative: Global IP Europe Patentanwaltskanzlei
(86) International application number: PCT/JP2022/043673
(87) International publication number: WO 2023/100783

(57) **Abstract**

When a disease is discriminated using principal component analysis, the accuracy of discriminating a predetermined disease is higher than that of conventional clinical markers, but the accuracy is still insufficient, and higher accuracy is required. The method for examining a disease or health condition includes a first step, a second step, a third step, and a fourth step. The first step extracts a biomarker (50) from a biological sample (40). The second step ionizes the extracted biomarker (50). The third step obtains a three-dimensional profile (22) by passing the ionized biomarker (50) between two electrodes and providing the two electrodes with a plurality of electrical signals. The fourth step discriminates a predetermined disease using a discriminator (342) obtained by learning the three-dimensional profile (22) and the type of disease in association with each other.

## Description

### TECHNICAL FIELD

The present invention relates to disease or health condition examination method and examination system.

### BACKGROUND ART

As described in Non Patent Literature 1, a method for discriminating a predetermined disease on the basis of biomarkers in a biological sample is known.

### SUMMARY OF INVENTION

### <Technical Problem>

As in Non Patent Literature 1, when a disease is discriminated using principal component analysis, the accuracy of discriminating a predetermined disease is higher than that of conventional clinical markers, but the accuracy is still insufficient, and higher accuracy is required.

### <Solution to Problem>

A method for examining a disease or health condition according to a first aspect is a method for examining a disease or health condition based on a biomarker in a biological sample. The method for examining a disease or health condition includes a first step, a second step, a third step, and a fourth step. The first step extracts a biomarker from a biological sample. The second step ionizes the extracted biomarker. The third step obtains a three-dimensional profile by passing the ionized biomarker between two electrodes and providing the two electrodes with a plurality of electrical signals. The fourth step discriminates a predetermined disease using a discriminator obtained by learning the three-dimensional profile and the type of disease in association with each other.

The method for examining a disease or health condition according to the first aspect can discriminate a predetermined disease with higher accuracy by using a discriminator obtained by learning the three-dimensional profile and the type of disease in association with each other.

A method for examining a disease or health condition according to a second aspect is the method for examining a disease or health condition according to the first aspect, in which the discriminator simultaneously discriminates multiple types of diseases.

A method for examining a disease or health condition according to a third aspect is the method for examining a disease or health condition according to the first aspect or the second aspect, in which the biological sample is at least one of exhaled breath, serum, blood, saliva, plasma, nipple aspiration material, synovial fluid, cerebrospinal fluid, sweat, urine, feces, tears, gas emitted from skin, tracheal washes, swabbed material, needle aspiration material, semen, vaginal fluid, and pre-ejaculate.

A method for examining a disease or health condition according to a fourth aspect is the method for examining a disease or health condition according to any one of the first to third aspects, in which the biological sample is exhaled breath or urine.

A method for examining a disease or health condition according to a fifth aspect is the method for examining a disease or health condition according to any one of the first to fourth aspects, in which the biomarker is at least one of a gene biomarker, a cell biomarker, an organic compound biomarker, a metabolic biomarker, a saccharide biomarker, a lipid biomarker, a heterocyclic biomarker, an elemental compound biomarker, an image biomarker, an anthropological biomarker, a personal habit biomarker, a disease state biomarker, and an expression biomarker.

A method for examining a disease or health condition according to a sixth aspect is the method for examining a disease or health condition according to any one of the first to fifth aspects, in which the biomarker is an organic compound biomarker generated for metabolism in vivo.

A method for examining a disease or health condition according to a seventh aspect is the method for examining a disease or health condition according to the sixth aspect, in which the organic compound biomarker is a volatile organic compound.

A method for examining a disease or health condition according to an eighth aspect is the method for examining a disease or health condition according to the seventh aspect, in which the volatile organic compound is volatilome.

A method for examining a disease or health condition according to a ninth aspect is the method for examining a disease or health condition according to any one of the first to eighth aspects, in which the discriminator discriminates non-disease, mild cancer, and severe cancer.

A method for examining a disease or health condition according to a tenth aspect is the method for examining a disease or health condition according to any one of the first to ninth aspects, in which the third step obtains the three-dimensional profile by utilizing a fact that mobility of ions changes between a low electric field and a high electric field.

An examination system for a disease or health condition according to an eleventh aspect is an examination system for a disease or health condition based on a biomarker in a biological sample. The examination system for a disease or health condition includes a first device, a second device, and a third device. The first device extracts a biomarker from a biological sample. The second device ionizes the extracted biomarker. The second device obtains a three-dimensional profile by passing the ionized biomarker between two electrodes and providing the two electrodes with a plurality of electrical signals. The third device discriminates a predetermined disease using a discriminator obtained by learning the three-dimensional profile and the type of disease in association with each other.

The examination system for a disease or health condition according to the eleventh aspect can discriminate a predetermined disease with higher accuracy by using the discriminator obtained by learning the three-dimensional profile and the type of disease in association with each other.

### BRIEF DESCRIPTION OF DRAWINGS

FIG. 1 is a configuration diagram of an examination system.
FIG. 2 is a diagram illustrating an example of a FAIMS profile.
FIG. 3 is a diagram for comparing FAIMS profiles between a patient with a disease and a healthy person.
FIG. 4 is a control block diagram of a discrimination device.
FIG. 5 is a diagram illustrating a configuration of a discriminator.
FIG. 6 is a diagram illustrating a discrimination result by principal component analysis.
FIG. 7 is a diagram illustrating a discrimination result by the discriminator.
FIG. 8 is an explanatory flowchart of an example of processing of the examination system.

### DESCRIPTION OF EMBODIMENTS

### (1) Overall configuration

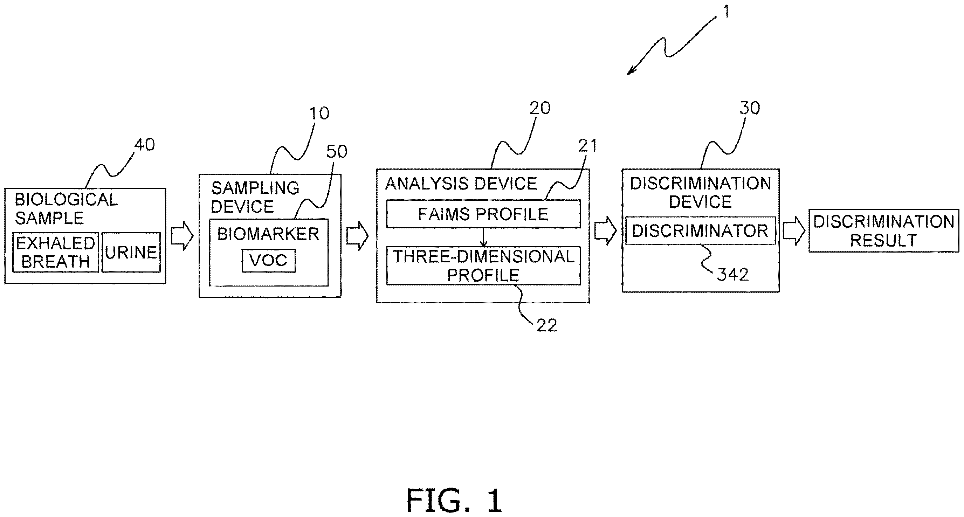

FIG. 1 is a configuration diagram of an examination system 1 for a disease or health condition. The examination system 1 discriminates a predetermined disease on the basis of a biomarker 50 in a biological sample 40.

As illustrated in FIG. 1, the examination system 1 includes a sampling device 10 (first device), an analysis device 20 (second device), and a discrimination device 30 (third device).

### (2) Detailed Configuration

### (2-1) Sampling device

The sampling device 10 extracts the biomarker 50 from the biological sample 40.

The biological sample 40 is at least one of exhaled breath, serum, blood, saliva, plasma, nipple aspiration material, synovial fluid, cerebrospinal fluid, sweat, urine, feces, tears, gas emitted from skin, tracheal washes, swabbed material, needle aspiration material, semen, vaginal fluid, and pre-ejaculate. The biological sample 40 in the present embodiment is exhaled breath or urine.

The biomarker 50 is at least one of a gene biomarker, a cell biomarker, an organic compound biomarker, a metabolic biomarker, a saccharide biomarker, a lipid biomarker, a heterocyclic biomarker, an elemental compound biomarker, an image biomarker, an anthropological biomarker, a personal habit biomarker, a disease state biomarker, and an expression biomarker. The biomarker 50 in the present embodiment is an organic compound biomarker generated for metabolism in vivo. Furthermore, in the present embodiment, VOC (Volatile Organic Compound) is used as the organic compound biomarker. In other words, the VOC in the present embodiment is volatilome (volatile metabolite).

As illustrated in FIG. 1, the sampling device 10 adsorbs VOC in the biological sample 40 to an adsorbent, thereby extracting VOC from the biological sample 40. Then, the sampling device 10 desorbs the VOC from the adsorbent, and uniformly (stably) flows the VOC into the analysis device 20 for a certain period of time.

### (2-2) Analysis device

The analysis device 20 analyzes the VOC extracted from the biological sample 40 by the sampling device 10 using FAIMS (Field Asymmetric Ion Mobility Spectrometry, High-Field Asymmetric Waveform Ion Mobility Spectrometry). The analysis device 20 is, for example, Lonestar from Owlstone.

FAIMS is an analysis technique utilizing the fact that mobility of ions changes between a low electric field and a high electric field.

The analysis device 20 includes a processor such as a CPU or a GPU, and a storage device such as a RAM, a ROM, or an HDD. In addition, the analysis device 20 includes a keyboard and a mouse for inputting various commands and various information. Further, the analysis device 20 includes a monitor for displaying a FAIMS profile 21 and the like to be described later. Furthermore, the analysis device 20 includes a network interface device for communicating with the discrimination device 30 via a communication line (not illustrated).

The analysis device 20 first ionizes the VOC extracted from the biological sample 40 by the sampling device 10. The analysis device 20 then generates the FAIMS profile 21 by passing the ionized VOC between two electrodes and providing the two electrodes with a plurality of electrical signals.

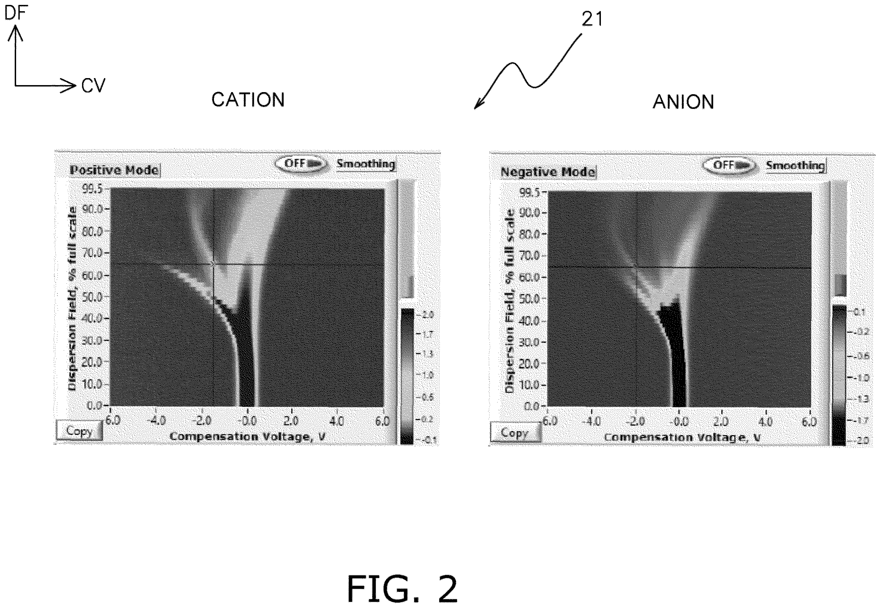

The FAIMS profile 21 records current values of cations and current values of anions flowing between two electrodes for each combination of a dispersion field (DF) and a compensation voltage (CV). FIG. 2 is a diagram illustrating an example of the FAIMS profile 21. In FIG. 2, the FAIMS profile 21 is represented by a two-dimensional array in which the first dimension (vertical axis) is the dispersion field (hereinafter, may be referred to as DF.) and the second dimension (horizontal axis) is the compensation voltage (hereinafter, may be referred to as CV). The left diagram of FIG. 2 is a FAIMS profile 21 recording the current values of cations (hereinafter, may be referred to as the FAIMS profile 21 of cations.), and the right diagram of FIG. 2 is a FAIMS profile 21 recording the current values of anions (hereinafter, may be referred to as the FAIMS profile 21 of anions.). In FIG. 2, the DF of "0% to 100%" is divided into 51 stages, and the CV of "-6 V to +6 V" is divided into 512 stages. In other words, the FAIMS profiles 21 of cations and anions in FIG. 2 are a two-dimensional array having a size of "51 × 512", respectively. In FIG. 2, the magnitude of the current value of each cell of the FAIMS profile 21 is expressed by the shade of color.

When the biological sample 40 is collected from a patient with a disease and a healthy person, it is known that the concentrations of specific substances contained in the VOC extracted from the biological sample 40 is different between the patient and the healthy person.

For example, Non Patent Literature 2 shows that when exhaled breath is collected from a patient with colorectal cancer and a healthy person, the concentrations of substances in the following Table 1 contained in VOCs extracted from the exhaled breath are different between the patient and the healthy person.

**[Table 1]**

| Substances in VOCs with different concentrations |
|---|
| Ethanol |
| Acetic acid |
| Methylbenzene |
| Dimethyl heptane |
| Hexanal |
| Octane, 4-methyl |
| Butanoic acid |
| Ethylbenzene |
| Xylene |
| 2-Butoxy ethanol |
| Decane |
| Benzaldehyde |
| Octanal |
| Undecane |
| 2-Ethyl-1-hexanol |
| 3,3-Dimethyl octane |
| Nonanal |
| Dodecane |
| Decanal |
| Benzoic acid |
| 4,6-Dimethyl dodecane |
| Tridecane |
| Benzene, 1,3-bis(1-methylethenyl) |
| Nonanoic acid |
| Tetradecane |
| 2,4,4,6,6,8,8-Heptamethyl-1-nonene |
| Decanoic acid |
| Ethanone, 1-[4-(1-methyl-ethenyl)-phenyl] |
| Pentadecane |
| Nonadecane |
| 5,9-Undecadien-2-one, 6,10-dimethyl |
| Butyl hydroxy toluene |

In addition, Non Patent Literature 3 shows that when exhaled breath is collected from a patient with colorectal cancer and a healthy person, the concentrations of substances in the following Table 2 contained in VOCs extracted from the exhaled breath are different between the patient and the healthy person.

**[Table 2]**

| Substances in VOCs with different concentrations |
|---|
| NonaA23:A38 |
| 4-Methyl-2-pentanone |
| Decanal |
| 2-Methylbutane |
| 1,2-Pentadiene |
| 2-Methylpentane |
| 3-Methylpentane |
| Methylcyclopentane |
| Cyclohexane |
| Methylcyclohexane |
| 1,3-Dimethylbenzene |
| 4-Methyloctane |
| 1,4-Dimethylbenzene |
| 4-methylundecane |
| Trimethyldecane |

In addition, Non Patent Literature 4 shows that when urine is collected from a patient with colorectal cancer and a healthy person, the concentrations of substances in the following Table 3 contained in VOCs extracted from the urine are different between the patient and the healthy person.

**[Table 3]**

| Substances in VOCs with different concentrations |
|---|
| Hexanal |
| Hexen-1-ol |
| 4-methyl-1 -hexene |
| Acetaldehyde |
| Ethylene Oxide |
| Oxalic Acid |
| Dimethyl Diazene |
| Cyclobutyl Amine |
| Oxepane |
| Acetone |
| 2-Pentanone |
| 3-methyl-2-Butanone |
| 2,3-Butanedione |
| 4-Heptanone |
| 3-Heptanone |
| 2,4-dimethyl-3-Pentanone |
| Acetyloxime-Pyridine Carboxaldehyde |
| Hydrocinnamoyl-Bezene-ethanamine |
| Styrene |
| Dimethyl-Thiourea |
| Allyl Isothiocyanate |
| Isothiocyanato-cyclopropane |
| 2-cyano-acetamide |
| Methoxy-phenyl-oxime |
| Ethylbenzoic acid (pentyl ester) |
| Carbamic acid (methyl ester) |

In addition, Non Patent Literature 5 shows that when urine is collected from a patient with colorectal cancer and a healthy person, the concentrations of substances in the following Table 4 contained in VOCs extracted from the urine are different between the patient and the healthy person.

**[Table 4]**

| Substances in VOCs with different concentrations |
|---|
| Octane, 2,2,6-trimethyl |
| Ethylbenzene |
| Xylene |
| Octanal |
| Undecanal |
| NonaA23:A38 |
| Decanal |
| Benzene, 1,3-bis(1-methylethenyl) |
| 1-Undecanol |
| Acetone |
| 2-Pentanone |
| 2-Heptanone |
| 2,4-Di-tert-butylphenol |
| Heptanal |
| Heptadecane |
| 3,4-Dimethylcyclohexanol |
| 5-Hepten-2-ol |
| 6-methyl-Hepten-2-ol |
| Hexanal |
| Biphenyl |
| Cyclopentanone, 2-methyl |
| Methane, isocyanato |
| Acetophenone |
| Naphthalene |

In addition, Non Patent Literature 6 shows that when exhaled breath is collected from a patient with liver cancer and a healthy person, the concentrations of substances in the following Table 5 contained in VOCs extracted from the exhaled breath are different between the patient and the healthy person.

**[Table 5]**

| Substances in VOCs with different concentrations |
|---|
| Ethane |
| Acetaldehyde |
| (E)-2-Nonene |
| Acetone |
| Trimethylamine |
| Propanol |
| Methylhexane |
| Acrylonitrile |
| Benzene |
| Dimethyl Sulfide |
| Hydrogen Sulfide |
| Isoprene |
| Ethanol |
| Octene |
| Carbon Disulfide |
| Ammonia |
| Nonene |
| Pentane |
| Heptane |
| Decene |
| Acetonitrile |
| Triethyl Amine |

In addition, Non Patent Literature 7 shows that when exhaled breath is collected from a patient with pancreatic cancer and a healthy person, the concentrations of substances in the following Table 6 contained in VOCs extracted from the exhaled breath are different between the patient and the healthy person.

**[Table 6]**

| Substances in VOCs with different concentrations |
|---|
| Methanol |
| Acetonitrile |
| Ethanol |
| MEK |
| Acetone |
| Isoprene |
| n-Propanol |
| Benzene |
| Toluene |
| n-Heptane |

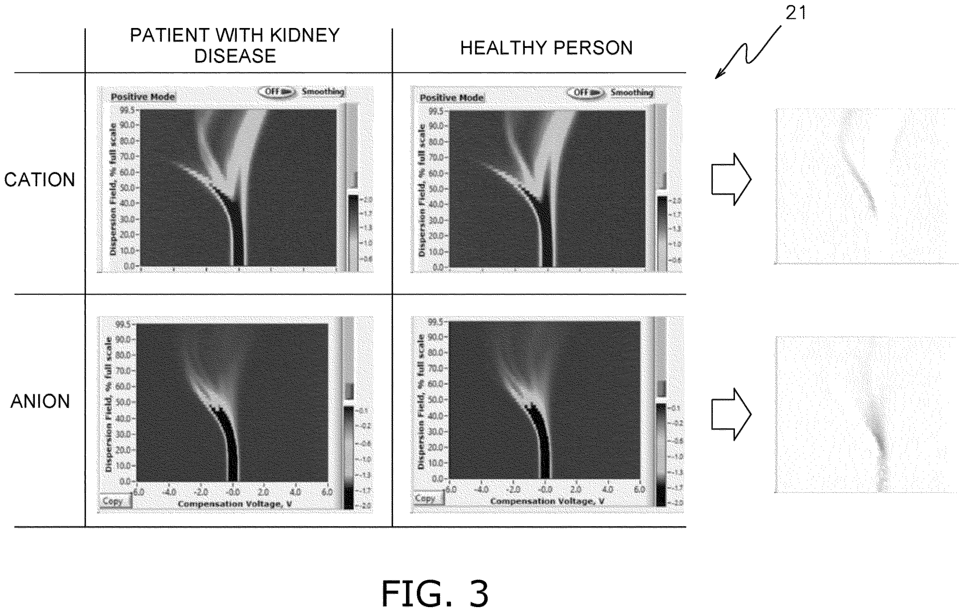

Such differences in the concentrations of specific substances contained in the VOCs affect the FAIMS profile 21. Specifically, the distribution of current values in the FAIMS profile 21 is different between the patient with a disease and the healthy person due to the difference in the concentrations of specific substances contained in the VOCs. FIG. 3 is a diagram for comparing FAIMS profiles 21 of the patient with a disease and a healthy person. In FIG. 3, using urine as a biological sample, the FAIMS profiles 21 of a patient with kidney disease and a healthy person are compared. In the region to the left of the arrow in FIG. 3, the left is the FAIMS profile 21 of the patient with kidney disease and the right is the FAIMS profile 21 of the healthy person. Also, the upper side is the FAIMS profile 21 of cations and the lower side is the FAIMS profile 21 of anions. In the region to the right of the arrow in FIG. 3, a diagram showing a difference in the FAIMS profiles 21 between the patient with kidney disease and the healthy person for each of the FAIMS profiles 21 of cations and anions is depicted. In the diagram showing the difference, the magnitude of the difference in the current value of each cell of the FAIMS profile 21 is expressed by the shade of color. In the diagram showing the difference, since the shade of color can be seen, it can be seen that the distribution of the current value of the FAIMS profile 21 is different between the patient with kidney disease and the healthy person.

Next, the analysis device 20 stacks the generated FAIMS profiles 21 of cations and anions in the direction of the third dimension to obtain a three-dimensional profile 22 (three-dimensional array). For example, in the case of FIG. 2, the analysis device 20 obtains a three-dimensional profile 22 having a size of "51 × 512 × 2" by stacking the FAIMS profiles 21 of cations and anions.

### (2-3) Discrimination device

The discrimination device 30 is a general computer. The discrimination device 30 discriminates a predetermined disease using a discriminator 342 obtained by learning the three-dimensional profile 22 and the type of disease in association with each other.

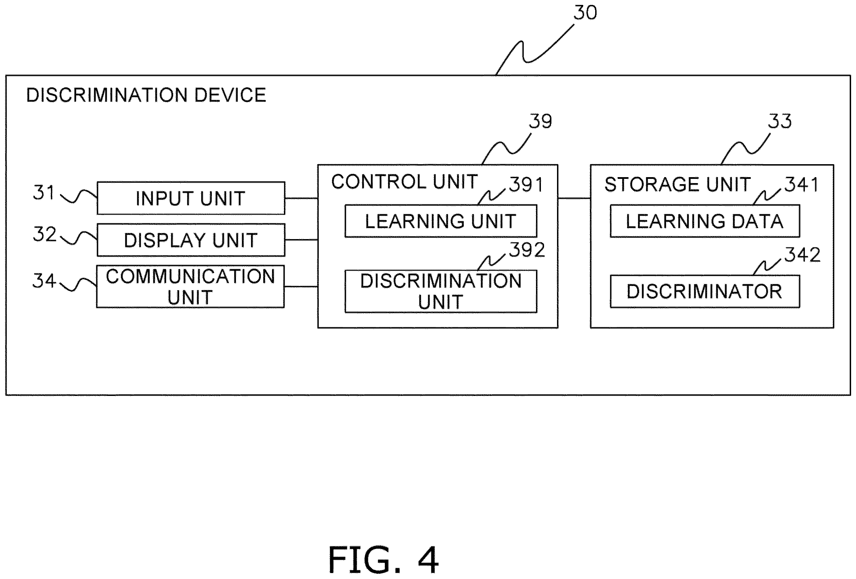

FIG. 4 is a control block diagram of the discrimination device 30. As illustrated in FIG. 4, the discrimination device 30 mainly includes an input unit 31, a display unit 32, a storage unit 33, a communication unit 34, and a control unit 39.

### (2-3-1) Input unit

The input unit 31 is a keyboard and a mouse. Various commands and various types of information for the discrimination device 30 can be input using the input unit 31.

### (2-3-2) Display unit

The display unit 32 is a monitor. The display unit 32 can display a learning situation and the like.

### (2-3-3) Communication unit

The communication unit 34 is a network interface device for communicating with the analysis device 20 via a communication line (not illustrated).

### (2-3-4) Storage unit

The storage unit 33 is a storage device such as a RAM, a ROM, and an HDD. The storage unit 33 stores a program implemented by the control unit 39, data necessary for implementing the program, and the like.

The storage unit 33 particularly stores learning data 341 and the discriminator 342.

The learning data 341 is data for performing learning of the discriminator 342. Specifically, the learning data 341 is data in which the three-dimensional profile 22 of VOC extracted from the biological sample 40 has been associated with the type of disease of a person from which the biological sample 40 has been collected. The three-dimensional profile 22 is obtained from the analysis device 20 via the communication unit 34, for example. The type of disease is input by a user from the input unit 31, for example.

### (2-3-5) Control unit

The control unit 39 is a processor such as a CPU or a GPU. The control unit 39 reads and executes the program stored in the storage unit 33 to implement various functions of the discrimination device 30. The control unit 39 can also write a calculation result to the storage unit 33 and read information stored in the storage unit 33 in accordance with the program.

The control unit 39 includes a learning unit 391 and a discrimination unit 392 as functional blocks.

### (2-3-5-1) Learning unit

The learning unit 391 performs learning of the discriminator 342 by using the learning data 341. In the present embodiment, it is assumed that the size of the three-dimensional profile 22 of the learning data 341 is "51 × 512 × 2". Furthermore, it is assumed that the learning data 341 is data in which the three-dimensional profile 22 has been associated with one of a "patient with a predetermined disease" and a "healthy person". In other words, the discriminator 342 discriminates whether it is a "patient with a predetermined disease" or a "healthy person" based on the three-dimensional profile 22. In addition, the discriminator 342 uses a model combining a convolutional neural network (CNN) and a recurrent neural network (RNN).

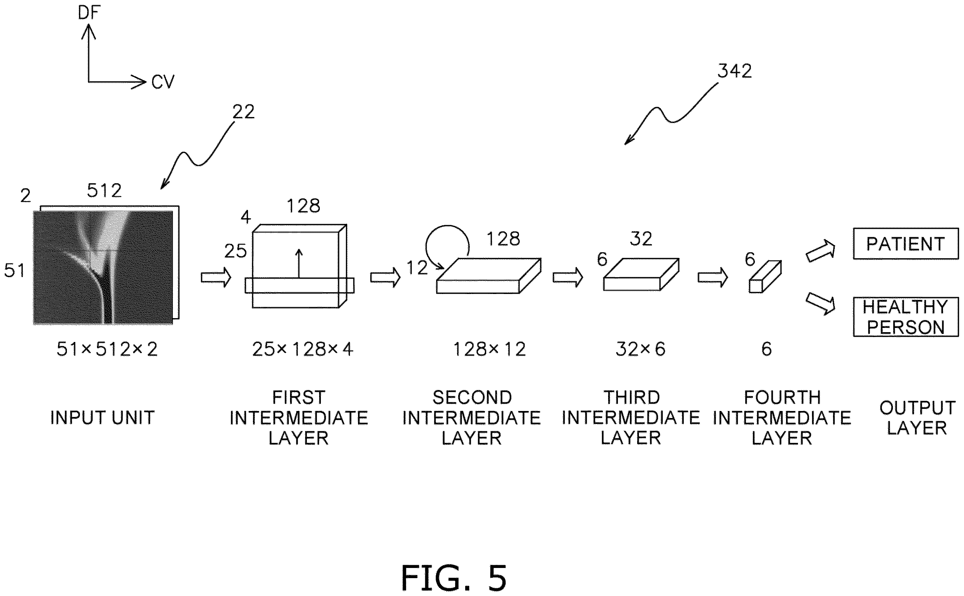

FIG. 5 is a diagram illustrating a configuration of the discriminator 342. As illustrated in FIG. 5, the discriminator 342 includes an input layer, first to fourth intermediate layers, and an output layer.

Between the input layer and the first intermediate layer, two-dimensional convolution (Conv2D) is performed on the dimensions of DF and CV. As a result, the size of the data flowing through the discriminator 342 is changed from "51 × 512 × 2" to "25 × 128 × 4".

Between the first intermediate layer and the second intermediate layer, the array is extracted row by row in ascending order of DF (25 times in total), and input to the RNN (Conv1D + RNN). In each step of the RNN, one-dimensional convolution is performed on the dimension of CV, and the dimension is reduced. Since the FAIMS profile 21 has continuity of current values in the dimension of DF, inputting into a recurrent layer (RNN) reduces the parameters and also improves the performance of the model. As a result, the size of the data flowing through the discriminator 342 changes from "25 × 128 × 4" to "128 × 12".

Between the second intermediate layer and the third intermediate layer, one-dimensional convolution (Conv1D) is performed on the dimension of CV. As a result, the size of the data flowing through the discriminator 342 changes from "128 × 12" to "32 × 6".

Between the third intermediate layer and the fourth intermediate layer, a dimension is reduced by average pooling. As a result, the size of the data flowing through the discriminator 342 changes from "32 × 6" to "6".

Between the fourth intermediate layer and the output layer, the probability of being a "patient with a predetermined disease" and the probability of being a "healthy person" are calculated by the fully connected layer. From the calculated probability, whether it is a "patient with a predetermined disease" or a "healthy person" is discriminated using a predetermined threshold value. As a result, the size of the data flowing through the discriminator 342 changes from "6" to "2".

### (2-3-5-2) Discrimination unit

The discrimination unit 392 discriminates a predetermined disease using the discriminator 342 learned by the learning unit 391. In the present embodiment, the discriminator 342 discriminates whether it is a "patient with a predetermined disease" or a "healthy person" from the three-dimensional profile 22.

### (3) Verification

In this verification, the discrimination accuracy between the conventional principal component analysis and the discriminator 342 was compared using urine collected from the "patient with a disease" and the "healthy person".

Both discrimination methods are the same until a three-dimensional profile 22 is obtained by the analysis device 20. In the principal component analysis, a three-dimensional profile 22 having a size of "51 × 512 × 2" was processed into a one-dimensional array having 52224 (= 51 × 512 × 2) elements. The discriminator 342 uses, as the learning data 341, data in which the three-dimensional profile 22 has been associated with one of the "patient with a disease" and the "healthy person".

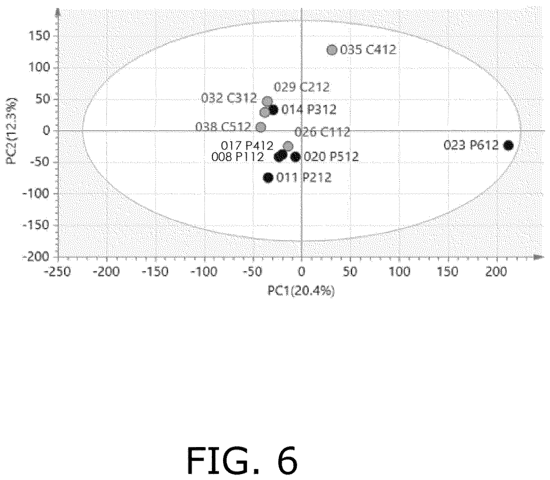

FIG. 6 is a diagram illustrating a discrimination result by principal component analysis. In FIG. 6, the "patient with a disease" and the "healthy person" are plotted on a two-dimensional eigenspace consisting of a first principal component PC1 and a second principal component PC2. The contribution ratio of the first principal component PC1 is 20.4%, and the contribution ratio of the second principal component PC2 is 12.3%. In FIG. 6, a relatively light-colored plot represents the "healthy person", and a relatively dark-colored plot represents the "patient with a disease". In the principal component analysis, as shown in FIG. 6, the analysis result showed that the "patient with a disease" and the "healthy person" were mixed. In general, in the principal component analysis, nonlinearity between features (current values) and contamination of a small number of unique sample data impair the representativeness of correlation coefficients, and distort principal components extracted thereafter. In addition, as shown in FIG. 6, when only the first principal component PC1 and the second principal component PC2 having a large influence on the sample data are plotted and discriminated, all features (current values) cannot be considered for discrimination in the principal component analysis, due to the influence of ignoring the remaining principal components, and the like. Therefore, it is difficult to discriminate between the "patient with a disease" and the "healthy person" by principal component analysis.

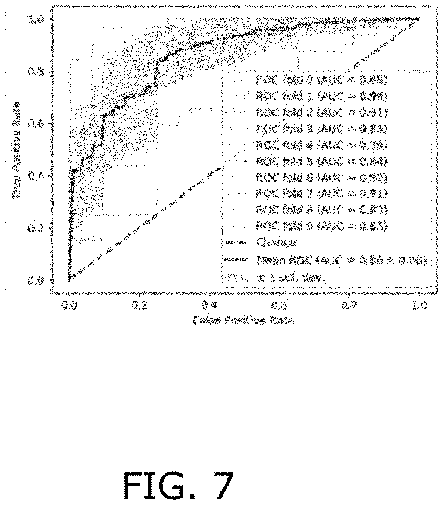

FIG. 7 is a diagram illustrating a discrimination result by the discriminator 342. FIG. 7 depicts ROC curves of each time when learning is performed by stratified 10-fold cross-validation and an average ROC curve of these curves. As illustrated in FIG. 7, since the AUC (Area Under the Curve) of the average ROC curve is 0.86 ± 0.08, it can be seen that the discriminator 342 discriminates between the "patient with a disease" and the "healthy person" with high accuracy. In the discriminator 342, all the features (current values) constituting the FAIMS profile 21 are considered for discrimination, so that the discrimination accuracy can be improved.

### (4) Processing

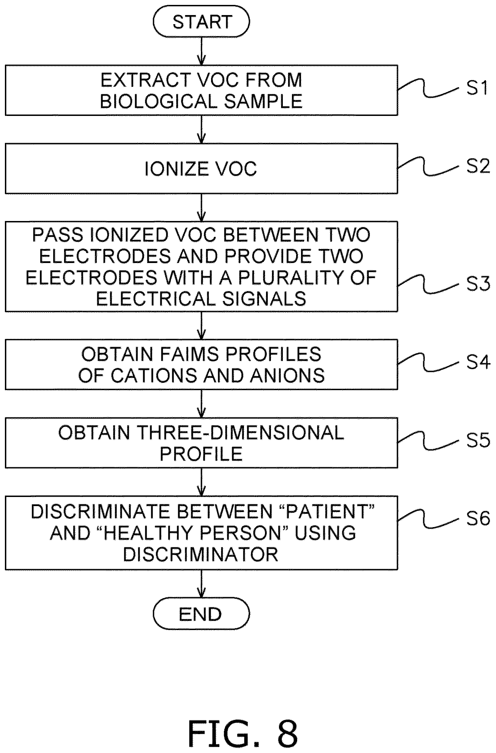

An example of processing of the examination system 1 will be described with reference to the flowchart of FIG. 8.

As shown in step S1, the sampling device 10 extracts VOC from the biological sample 40.

Upon completion of step S1, as shown in step S2, the analysis device 20 ionizes the VOC extracted from the biological sample 40.

Upon completion of step S2, as shown in step S3, the analysis device 20 passes the ionized VOC between two electrodes and provides the two electrodes with a plurality of electrical signals.

Upon completion of step S3, as shown in step S4, the analysis device 20 generates FAIMS profiles 21 of cations and anions.

Upon completion of step S4, as shown in step S5, the analysis device 20 stacks the FAIMS profiles 21 of cations and anions to obtain a three-dimensional profile 22.

Upon completion of step S5, as shown in step S6, the discrimination device 30 discriminates whether it is a "patient with a predetermined disease" or a "healthy person" using the discriminator 342 obtained by learning the three-dimensional profile 22 and the "patient with a predetermined disease" or the "healthy person" in association with each other.

### (5) Features

(5-1)
As described in Non Patent Literature 1, a method for discriminating a predetermined disease on the basis of biomarkers in a biological sample is known.

However, when a disease is discriminated using principal component analysis as in Non Patent Literature 1, the accuracy of discriminating a predetermined disease is higher than that of conventional clinical markers, but as shown by verification, the accuracy is still insufficient, and higher accuracy is required.

The examination system 1 of the present embodiment is an examination system of a disease or health condition based on a VOC (biomarker) in a biological sample 40. The examination system 1 includes a sampling device 10, an analysis device 20, and a discrimination device 30. The sampling device 10 extracts VOC from the biological sample 40. The analysis device 20 ionizes the extracted VOC. The analysis device 20 obtains a three-dimensional profile 22 by passing the ionized VOC between two electrodes and providing the two electrodes with a plurality of electrical signals. In particular, the analysis device 20 obtains the three-dimensional profile 22 using FAIMS. The discrimination device 30 discriminates a predetermined disease using a discriminator 342 obtained by learning the three-dimensional profile 22 and the type of disease in association with each other.

The examination system 1 can discriminate a predetermined disease with higher accuracy by using the discriminator 342 obtained by learning the three-dimensional profile 22 and the type of disease in association with each other.

### (6) Modification examples

### (6-1) Modification 1A

In the present embodiment, the learning data 341 was data in which the three-dimensional profile 22 had been associated with one of the "patient with a predetermined disease" and the "healthy person".

However, the learning data 341 may be data in which the three-dimensional profile 22 has been associated with multiple types of diseases. The learning data 341 is, for example, data in which the three-dimensional profile 22 has been associated with any of a "patient with colorectal cancer", a "patient with kidney cancer", and the "healthy person". At this time, the discriminator 342 can simultaneously discriminate multiple types of diseases by providing the number of nodes to be discriminated in the output layer.

### (6-2) Modification 1B

In the present embodiment, the learning data 341 was data in which the three-dimensional profile 22 had been associated with one of the "patient with a predetermined disease" and the "healthy person".

However, the learning data 341 may be data in which the three-dimensional profile 22 has been associated with any of "non-disease", "stage 1 cancer", "stage 2 cancer", "stage 3 cancer", and "stage 4 cancer". At this time, the discriminator 342 can discriminate "non-disease", "mild cancer (for example, cancers of stages 1 and 2)", and "severe cancer (for example, cancers of stages 3 and 4)" by providing the number of nodes to be discriminated in the output layer.

(6-3)
The embodiments of the present disclosure have been described above. It will be understood that various changes to modes and details can be made without departing from the gist and scope of the present disclosure recited in the claims.

### REFERENCE SIGNS LIST

1: Examination system
10: First device
20: Second device
22: Three-dimensional profile
30: Third device
342: Discriminator
40: Biological sample
50: Biomarker
S1: First step
S2: Second step
S3 to 5: Third step
S6: Fourth step

### CITATION LIST

### PATENT LITERATURE

Non Patent Literature 1: Christopher Psutka, Marina Yamada, Akihisa Matsuda, Kazuya Yamahatsu, Satoshi Matsumoto, Toshihiko Kitayama, Nobuo Nakano, Jyunichi Koyano, Tohru Mikoshiba, Masao Miyashita "FAIMS technology in urinary volatile organic compound analysis to detect colorectal cancer" AACR2017, Abstract No. 5303
Non Patent Literature 2: D. F. Altomare, A. Picciariello, M. T. Rotelli, M. DeFazio, A. Aresta, C. G. Zambonin, L. Vincenti, P. Trerotoli, N. De Vietro "Chemical signature of colorectal cancer: case-control study for profiling the breath print" BJS Open 2020; 4: 1189-1199
Non Patent Literature 3: D F Altomare, M Di Lena, F Porcelli, L Trizio, E Travaglio, M Tutino, S Dragonieri, V Memeo, G de Gennaro "Exhaled volatile organic compounds identify patients with colorectal cancer" British Journal of Surgery 2013; 100: 144-150
Non Patent Literature 4: Ramesh P Arasaradnam, Michael J McFarlane, Courtenay Ryan-Fisher, Erik Westenbrink, Phoebe Hodges, Matthew G Thomas, Samantha Chambers, Nicola O'Connell, Catherine Bailey, Christopher Harmston, Chuka U Nwokolo, Kama D Bardhan, James A Covington "Detection of colorectal cancer (CRC) by urinary volatile organic compound analysis" PLOS ONE 2014; 9: Issue 9 e108750
Non Patent Literature 5: Heena Tyagi, Emma Daulton, Ayman S. Bannaga, Ramesh P. Arasaradnam and James A. Covington "Non-Invasive Detection and Staging of Colorectal Cancer Using a Portable Electronic Nose" Sensors 2021; 21: 5440-
Non Patent Literature 6: Galen Miller-Atkins, Lou-Anne Acevedo-Moreno, David Grove, Raed A. Dweik, Adriano R. Tonelli, J. Mark Brown, Daniela S. Allende, Federico Aucejo, Daniel M. Rotroff "Breath Metabolomics Provides an Accurate and Noninvasive Approach for Screening Cirrhosis, Primary, and Secondary Liver Tumors" Hepatology Communications, Vol. 4, No. 7, 2020
Non Patent Literature 7: Andrea Princivalle, Lorenzo Monasta, Giovanni Butturini, Claudio Bassi & Luigi Perbellini "Pancreatic ductal adenocarcinoma can be detected by analysis of volatile organic compounds (VOCs) in alveolar air" BMC Cancer volume 18, Article number: 529 (2018)

## Claims

1. A method for examining a disease or health condition based on a biomarker (50) in a biological sample (40), the method comprising:
a first step (S 1) of extracting the biomarker from the biological sample;
a second step (S2) of ionizing the extracted biomarker;
a third step (S3 to 5) of obtaining a three-dimensional profile (22) by passing the ionized biomarker between two electrodes and providing the two electrodes with a plurality of electrical signals; and
a fourth step (S6) of discriminating a predetermined disease using a discriminator (342) obtained by learning the three-dimensional profile and a type of disease in association with each other.

2. The method for examining a disease or health condition according to claim 1, wherein the discriminator simultaneously discriminates multiple types of diseases.

3. The method for examining a disease or health condition according to claim 1 or 2, wherein the biological sample is at least one of exhaled breath, serum, blood, saliva, plasma, nipple aspiration material, synovial fluid, cerebrospinal fluid, sweat, urine, feces, tears, gas emitted from skin, tracheal washes, swabbed material, needle aspiration material, semen, vaginal fluid, and pre-ejaculate.

4. The method for examining a disease or health condition according to any one of claims 1 to 3, wherein the biological sample is exhaled breath or urine.

5. The method for examining a disease or health condition according to any one of claims 1 to 4, wherein the biomarker is at least one of a gene biomarker, a cell biomarker, an organic compound biomarker, a metabolic biomarker, a saccharide biomarker, a lipid biomarker, a heterocyclic biomarker, an elemental compound biomarker, an image biomarker, an anthropological biomarker, a personal habit biomarker, a disease state biomarker, and an expression biomarker.

6. The method for examining a disease or health condition according to any one of claims 1 to 5, wherein the biomarker is an organic compound biomarker generated for metabolism in vivo.

7. The method for examining a disease or health condition according to claim 6, wherein the organic compound biomarker is a volatile organic compound.

8. The method for examining a disease or health condition according to claim 7, wherein the volatile organic compound is volatilome.

9. The method for examining a disease or health condition according to any one of claims 1 to 8, wherein the discriminator discriminates non-disease, mild cancer, and severe cancer.

10. The method for examining a disease or health condition according to any one of claims 1 to 9, wherein the third step obtains the three-dimensional profile by utilizing a fact that mobility of ions changes between a low electric field and a high electric field.

11. An examination system (1) for a disease or health condition based on a biomarker (50) in a biological sample (40), the examination system (1) comprising:
a first device (10) configured to extract the biomarker from the biological sample;
a second device (20) configured to obtain a three-dimensional profile (22) by ionizing the extracted biomarker, passing the ionized biomarker between two electrodes, and providing the two electrodes with a plurality of electrical signals; and
a third device (30) configured to discriminate a predetermined disease using a discriminator (342) obtained by learning the three-dimensional profile and a type of disease in association with each other.
